# EUROPEAN PATENT SPECIFICATION

(11) **EP 3 389 576 B1**
(45) Date of publication and mention of the grant of the patent: **23.03.2022**
(21) Application number: 16819176.5
(22) Date of filing: 08.12.2016
(51) Int. Cl.: A61F 9/007, A61F 9/00

(54) **PATCH FOR SEALING RETINAL BREAKS AND ASSOCIATED DEVICES AND SYSTEMS.**
PATCH ZUR ABDICHTUNG VON NETZHAUTRISSEN UND ZUGEHÖRIGE VORRICHTUNGEN UND SYSTEME
TIMBRE DE SCELLEMENT DE DÉCOLLEMENTS DE LA RÉTINE ET DISPOSITIFS, ET SYSTÈMES

(30) Priority: 14.12.2015 US 201562266995 P
(43) Date of publication of application: 24.10.2018
(73) Proprietor: Alcon Inc., 1701 Fribourg (CH)
(72) Inventor: GUNN, Nicholas Max, Lake Forest CA 92630 (US); PAPAC, Michael J., Lake Forest CA 92630 (US); KASHANI, Pooria Sharif, Lake Forest CA 92630 (US)
(74) Representative: Murgitroyd & Company
(86) International application number: PCT/IB2016/057439
(87) International publication number: WO 2017/103744

(56) References cited:
- EP-A2- 2 853 236
- WO-A2-2008/082444
- US-A- 5 527 356
- US-A- 5 597 381
- US-A- 5 941 250
- US-A1- 2015 164 687

## Description

### TECHNICAL FIELD

The present disclosure is directed to ophthalmic surgical devices, systems, and methods. More particularly, but not by way of limitation, the present disclosure is directed to devices, systems, and methods of sealing a retinal break using a patch.

### BACKGROUND

Retinal breaks are physiological defects of the eye including holes or tears in the retina. Retinal breaks may cause vision impairment, and if left untreated, may lead to other, more serious physiological conditions such as retinal detachment and permanent vision loss. Ophthalmic microsurgical procedures are used to treat retinal breaks. Some surgical treatments for retinal breaks may include vitrectomy surgery, which involves the removal of vitreous humor from the vitreous chamber, followed by laser retinopexy/photocoagulation or cryopexy to create scar tissue around the retinal break. Over the course of several weeks, the scar tissue forms and securely holds the retina in position. In order for the scar tissue to form, the retinal break must be sealed to prevent the ingress of fluid under the retina. Currently, retinal breaks are sealed by injecting an oil, such as silicone oil, or a gas, such as sulfur hexafluoride (SF6) or octafluoropropane (CJFs), into the eye. Such procedures may be referenced as pneumatic retinopexy. When the patient's head is properly positioned, e.g., the patient is in a face-down position, the bubble of oil or gas presses against and seals the retinal break, which prevents fluid from going under or behind the retina. In some cases, the patient is required to hold their head face-down so that gas or oil bubble remains in the correct position throughout the many weeks required for scar tissue to form. When oil is used to seal the retinal break, the patient must undergo another surgical procedure to extract the oil from the eye after the scar tissue has formed. While the oil/gas tamponade procedure is typically efficacious in sealing retinal breaks, it is very inconvenient for the patient.

Reference is made to documents WO2008/082444, US5597381, US5527356, EP2853236 and US2015/164687 which have been cited as representative of the state of the art.

### SUMMARY

It will be appreciated that the scope of the invention is in accordance with the claims. Accordingly there is provided an ophthalmic surgical system as defined in claim 1 . Further features are provided in the dependent claims. The specification may include arrangements outside the scope of the claims and provided as background and to assist in understanding the invention. The specification includes description of the ophthalmic surgical methods 700 at paragraphs 0042 to 0048. The methods are not claimed and are provided as provided and for illustrative purposes.

According to one aspect, the present disclosure describes an ophthalmic surgical system including a patch sized and shaped to seal a retinal break of an eye by preventing fluid from infiltrating a sub-retinal space when adhered to the retina surrounding the retinal break. The system also includes a delivery device having a cannula configured to maintain the patch in a furled state for passage through an incision in the eye. The delivery device is actuatable to deploy the patch within a vitreous chamber of the eye.

Another aspect of the present disclosure is directed to an ophthalmic surgical system including a delivery device comprising a body, a cannula coupled to the body, and a shaft movably disposed within the cannula. The cannula may be sized and shaped to be inserted into a vitreous chamber of the eye. The system may also include a patch sized and shaped to seal a retinal break when adhered to the retina surrounding the retinal break. The patch may be disposed within the cannula in a furled state. Upon actuation of the shaft, the patch may be ejected from the cannula into the vitreous chamber, the patch being adjustable to an unfurled state.

A third aspect of the disclosure, not claimed, is directed to ophthalmic surgical method. The method (not being part of the invention) may include damaging tissue around a retinal break of an eye. The method may also include delivering, into the vitreous chamber of the eye, a patch sized and shaped to seal the retinal break. The method may also include positioning the patch on the retina surrounding the retinal break. The method may also include affixing the patch to the retina such that the patch prevents fluid from infiltrating a sub-retinal space.

The various aspects of the disclosure may include one or more of the following features. The patch may comprise at least one of a hydrogel, a biological material, and an elastomeric polymer. The system may further comprise an adhesive configured to affix the patch to the retina. The adhesive may be disposed on the patch. The adhesive may be activated upon exposure to liquid. The adhesive may be activated upon exposure to light. The system may further include an applicator sized and shaped for insertion into the vitreous chamber. The applicator may be configured to apply the adhesive to at least a perimeter of the patch while the patch is positioned on the retina. The delivery device may further comprise a body coupled to the cannula. The body may be sized and shaped for grasping by a user. The delivery device may include a shaft movably disposed within the cannula. The shaft may be configured to eject the patch from the cannula upon movement of the shaft. The shaft may be coupled to an actuation control controlling movement of the shaft. The actuation control may be disposed on the body of the delivery device.

The various aspects of the disclosure may also include one or more of the following features. The method may further include obtaining a delivery device including the patch in a furled state. Delivering the patch may include ejecting the patch from the delivery device in an unfurled state. Delivering the patch may include actuating a shaft of a delivery device. The delivery device may include a body, a cannula coupled to the body, and the shaft movably disposed within the cannula. The cannula may be sized and shaped to be inserted into the vitreous chamber. Actuating the shaft may eject the patch from the cannula. Affixing the patch to the retina may include applying an adhesive around a perimeter of the patch. Affixing the patch to the retina may include activating an adhesive disposed on the patch. Activating the adhesive may include exposing the adhesive to a liquid. Activating the adhesive may include exposing the adhesive to light.

It is to be understood that both the foregoing general description and the following drawings and detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following.

### BRIEF DESCRIPTION OF THE DRAWINGS

The accompanying drawings illustrate embodiments of the systems, devices, and methods disclosed herein and together with the description, serve to explain the principles of the present disclosure.
FIG. 1 is an illustration of an example ophthalmic surgical system.
FIG. 2 is a top or a bottom view of an example implementation of a patch of the ophthalmic surgical system of FIG. 1.
FIG. 3 is a side view of an example implementation of a patch of the ophthalmic surgical system of FIG. 1.
FIG. 4 is a side view of example alternative patch of the ophthalmic surgical system of FIG. 1.
FIGs. 5A, 5B, 5C, and 5D are illustrations showing example functionality of a delivery device of the ophthalmic surgical system of FIG. 1.
FIGs. 6A, 6B, 6C, 6D, and 6E are illustrations showing portions of the ophthalmic surgical system of FIG. 1 in situ in the eye.
FIG. 7 is a flow diagram of an example ophthalmic surgical method.

These figures will be better understood by reference to the following Detailed Description.

### DETAILED DESCRIPTION

For the purposes of promoting an understanding of the principles of the present disclosure, reference will now be made to the implementations illustrated in the drawings and specific language will be used to describe them. It will nevertheless be understood that no limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with reference to one or more implementations may be combined with the features, components, and/or steps described with reference to other implementations of the present disclosure. For simplicity, in some instances the same reference numbers are used throughout the drawings to refer to the same or like parts.

The present disclosure relates generally to devices, systems, and methods for sealing a retinal break using a biocompatible patch. The patch is sized and shaped to surround and cover the retinal break so that fluid does not infiltrate a sub-retinal space. A user, such as a surgeon or other medical professional, injects the patch into a patient's eye using a delivery device. For example, the delivery device may include a cannula in which the patch is positioned. Upon actuation of a control mechanism, a shaft within the cannula ejects the patch into the eye. An adhesive affixes the patch to the retina.

The devices, systems, and methods of the present disclosure provide numerous advantages over conventional systems. In particular, using the patch may replace the long and inconvenient gas/oil tamponade process that is conventionally used to hold the retina in place during the healing process for repairing retinal tears. Using the patch also eliminates the need for the patient to hold their head in face-down position during the multi-week healing period, greatly improving patient convenience. In some cases, the patient and the surgeon are also able to avoid the risk and inconvenience associated with an additional surgical procedure to remove oil from the patient's eye.

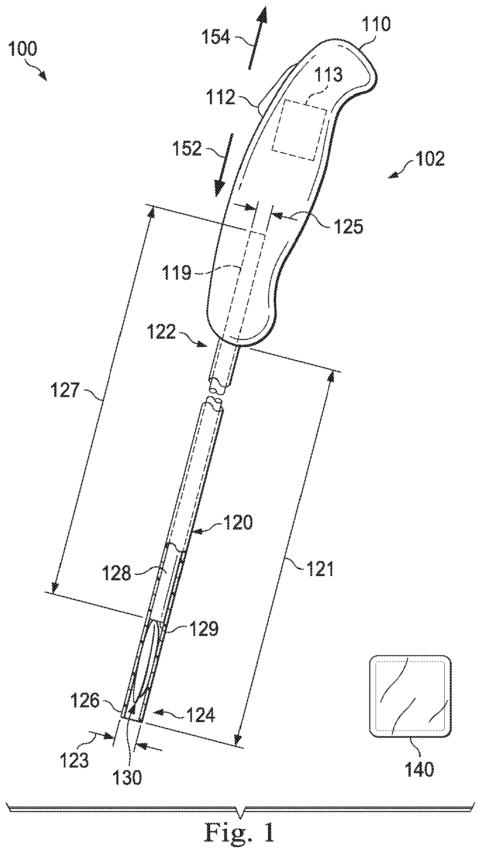

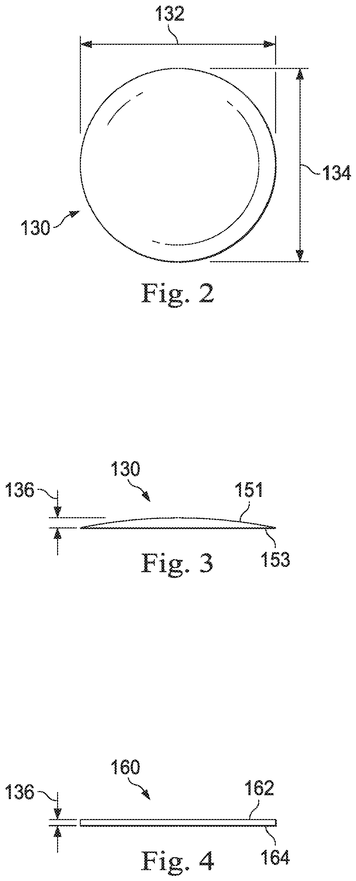

FIG. 1 illustrates an example ophthalmic surgical system 100. The system includes a delivery device 102, a patch 130, and an adhesive 140. Exemplary embodiments of the patch 130 are also illustrated in FIGs. 2-4. The patch 130 is configured to seal a break, tear, hole, and/or other abnormality in a retina such that fluid, such as vitreous humor, saline, gas, etc., does not infiltrate a sub-retinal space. Fluid in the sub-retinal space may have adverse physiological impacts on the patient, including delayed healing after laser retinopexy/photocoagulation or cryopexy, retinal detachment, and impaired vision.

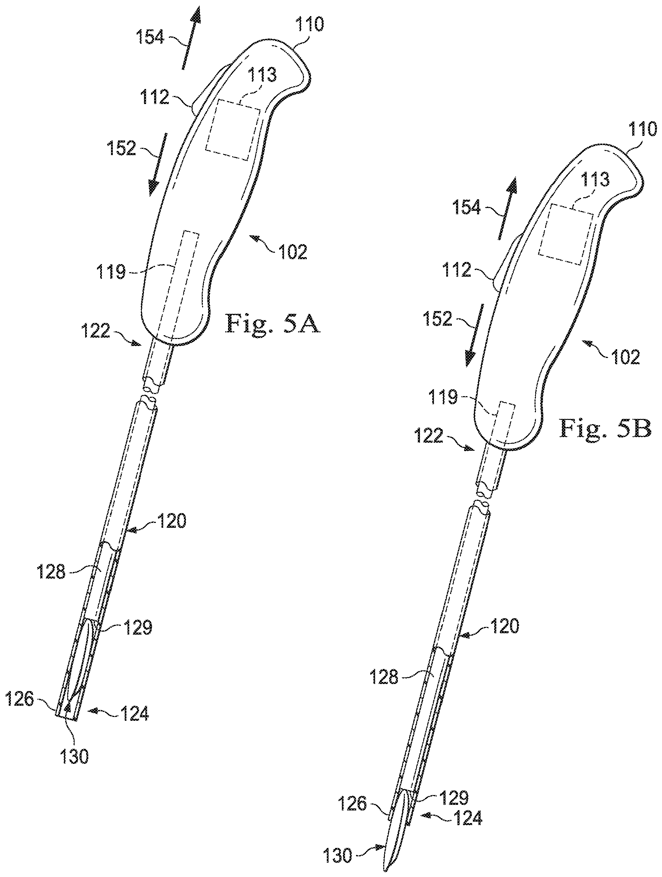

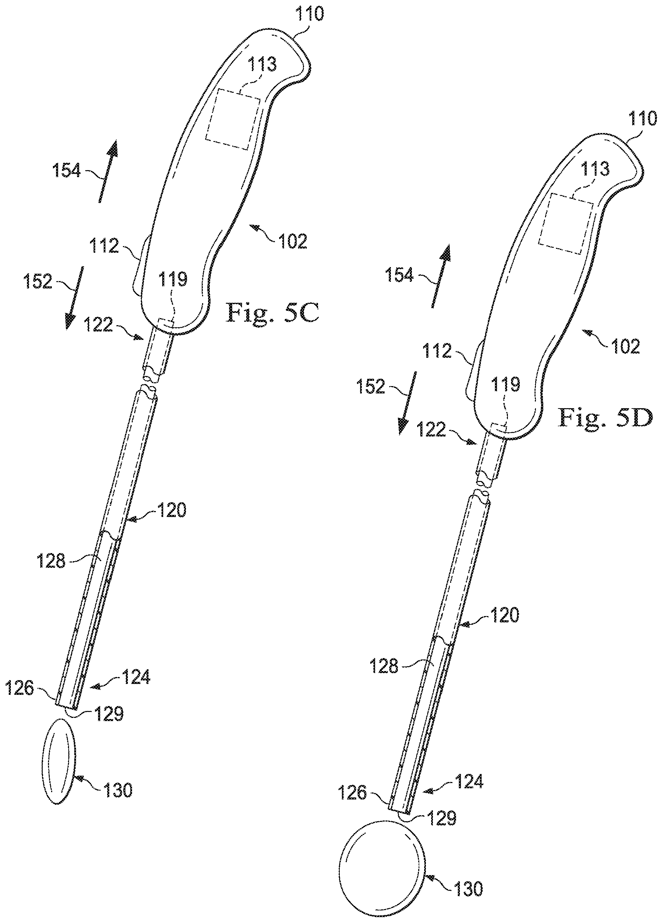

The patch 130 may be formed of a flexible material. For example, the patch 130 may be rolled, folded, pressed together, and/or otherwise furled to be smaller than when the patch 130 is in an unfurled state. FIGs. 1 and 5A show the patch 130 in a furled state and positioned within a cannula 120 of the delivery device 102. For example, the patch 130 may be folded two, three, four, or more times while disposed within the cannula 120. The patch 130 may become unrolled, unfolded, and/or otherwise unfurled when the patch 130 is ejected from the cannula 120 and deployed within the eye. For example, the patch 130 may be biased towards an unfurled state such that the patch 130 unrolls, unfolds, and/or otherwise becomes unfurled when the cannula 120 no longer prevents the patch 130 from doing so. FIGs. 5B and 5C show the patch 130 as it is being ejected from the cannula 120 and returning to an unfurled state. FIGs. 2, 3, and 5D illustrate an implementation of the patch 130 in an unfurled state. FIG. 4 shows an alternative embodiment of a patch, referenced herein as a patch 160.

Referring again to FIGs. 1-3, the patch 130 may be formed of one or more biocompatible materials. In some implementations, the patch 130 may be formed of a hydrogel, such as PEG-based hydrogel (poly(ethylene glycol)), polyvinyl alcohol-based hydrogel material, albumin-based hydrogel, acrylates, other suitable materials, and/or combinations thereof. In some implementations, the patch 130 may be formed of a biological material, such as a protein-based material including collagen, fibronectin, laminin, albumin, dextran, silk, fibrion, a material including an extra-cellular matrix, other suitable materials, and/or combinations thereof. In some implementations, the patch 130 may be formed of any synthetic and/or plastic materials, including parylene, polystyrene, polytetrafluorethylene (PTFE), thermamox (TPX), polyvinylchloride (PVC), polycarbonate, other plastics, other suitable materials, and/or combinations thereof. In some implementations, the patch 130 may be formed of any suitable elastomeric polymer. In some implementations, the patch 130 may include a substrate and a material disposed on the substrate. The substrate may be formed of collagen, silk (fibrion), parylene, polystyrene, polytetrafluorethylene (PTFE), polyethylene (PE), thermamox (TPX), polyvinylchloride (PVC), polycarbonate, other plastics, other suitable materials, and/or combinations thereof. The material disposed on the substrate may include a prepared biological such as collagen matrix, collagen, fibronectin, laminin, silk (fibrion), other suitable materials, and/or combinations thereof. Any suitable process may be used to dispose the material on the substrate, including physical vapor deposition, chemical vapor deposition, chemical adsorption, physical adsorption, dip coating, solvent evaporation, and/or other suitable processes.

In some implementations, the patch 130 is formed of a biodegradable material. For example, after being positioned within the eye, the patch 130 may degrade, break down, and/or be absorbed by the body after a period of time. The period of time may be one, two, three, four, or more weeks and include sufficient time for scar tissue to form around the retinal break. In some implementations, the patch 130 is formed of a material that does not break down or degrade over time. In that regard, the patch 130 may be permanently positioned around the retinal break. In such implementations, the surgeon may skip forming scar tissue around the retinal break as a therapeutic step because the patch 130 will be permanently positioned around the retinal break. The patch 130 may be colorless, clear, and/or translucent in some implementations.

The patch 130 may be sized and shaped in any of a variety of different sizes and shapes, depending upon the particular embodiment. A manufacturer may produce the different patches with different sizes, shapes, materials, and/or other parameters based on different therapeutic needs for different patients. For example, patches having different parameters may be used for retinal holes, retinal tears, giant retinal tears, different sized/shaped retinal breaks, and/or other physiology of the patient. In the illustrated implementations, the patch 130 is substantially circular, though in other implementations, the patch 130 may be polygonal, ellipsoidal, combinations thereof, and/or otherwise suitably shaped.

FIGs. 3 and 4 are side views of different implementations of the patch. Patch 160 of FIG. 4 is substantially similar to the patch 130 of FIGs. 1 and 2, and 3. The patch 130 includes faces 151, 153, and the patch 160 includes faces 162, 164. One of the faces 151, 153 of the patch 130 may be an anterior side that is exposed to the vitreous chamber, while the other of the faces 151, 153 may be a posterior side that is arranged to be proximate to and abut against the retina. Similarly, one of the faces 162, 164 may be the anterior side, while the other of the faces 162, 164 of the patch 160 may be the posterior side. The faces 151, 153, 162, 164 may be shaped in a variety of different ways depending upon the particular embodiment. For example, FIG. 3 illustrates that the face 151 of the patch 130 is substantially convex, while the face 153 is substantially planar. In that regard, the two faces 151, 153 of the patch 130 may be differently shaped. The patch 160 in FIG. 4 includes faces 162, 164 shaped to be substantially planar. In that regard, the two faces 162, 164 may be similarly shaped. In some implementations, either face may be the anterior side and the posterior side. In various implementations, the faces 151, 153, 162, 164 may be concave, convex, and/or otherwise curved, planar, flat, other suitable shapes, and/or combinations thereof. In some implementations, the faces 151, 153, 162, 164 may include grooves, projections, gratings, undulations, patterns, other suitable textures, and/or combinations thereof.

As shown in FIGs. 2-4, the patches (e.g., the patch 130, 160) may have dimensions 132, 134, 136. Dimensions 132, 134 may be a length, a width, and/or a diameter of the patches 130, 160. One or both of the dimensions 132, 134 may be between approximately 1 mm (millimeter) and approximately 15 mm, between approximately 1 mm and approximately 12 mm, between approximately 1 mm and approximately 10 mm, and/or other suitable values both larger and smaller. In some implementations, the dimensions 132, 134 are substantially similar, while in other implementations, the dimensions 132, 134 are different. Dimension 136 may be a height of the patch. The dimension 136 may be between approximately 0.1 mm and approximately 5 mm, between approximately 0.1 mm and approximately 3 mm, between approximately 0.1 mm and approximately 2 mm, and/or other suitable values both larger and smaller.

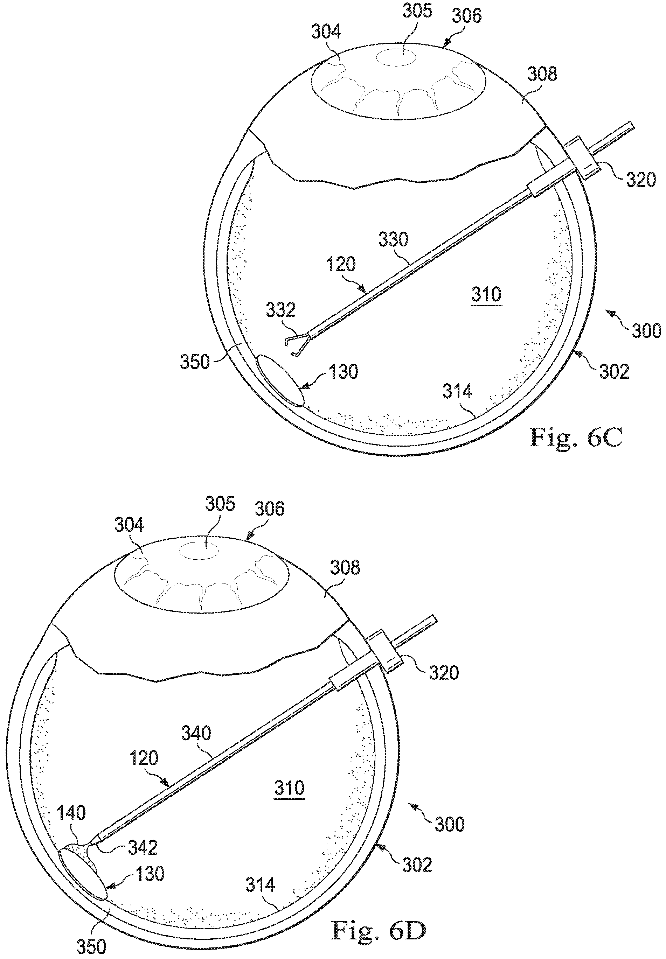

Referring again to FIG. 1, the adhesive 140 is configured to affix the patch 130 to anatomy within the eye, such as the retina. The adhesive 140 is a biocompatible material. Examples of the adhesive 140 include cyanoacrylate, PEG-based adhesives, polyvinyl alcohol-based adhesives, albumin-based adhesives, acrylate-based adhesives, light-activated adhesives, and/other suitable adhesives. In some implementations, the adhesive 140 and/or the patch 130 includes a cross-linking agent, such as an NHS (N-Hydroxysuccinimide) ester reagent, amine group, aldehyde, and/or other suitable materials or said materials attached to a polyethylene glycol (PEG) backbone. In some implementations, the adhesive 140 contains a first cross-linking agent reactive to a second cross-linking that is included in patch 130. In some implementations, the adhesive 140 and/or the patch 130 includes a curing agent, such as cyanoacrylate, and/or other suitable materials. In some implementations, the adhesive 140 is distinct and separate from the patch 130 until the adhesive is introduced onto the patch 130 and/or anatomy within the eye. In these types of implementations, the adhesive 140 may be stored in a container spaced or separate from the patch 130. For example, an applicator 340 (FIG. 6D) may be used to gather adhesive 140 from the container and deliver the adhesive 140 onto the retina and/or the patch 130. In some implementations, the adhesive 140 is disposed on or within, and/or otherwise integrated with the patch 130. For example, the adhesive 140 may be positioned on an outside of the patch (e.g., one or more of the faces 151, 153, 162, 164 of FIGs. 3 and 4). In some implementations, the patch 130 may be porous so that the adhesive 140 may be held within an interior of the patch 130, and may adhere the patch 130 to tissue. The adhesive 140 may be evenly or unevenly distributed on and/or within the patch 130. In some implementations, the adhesive 140 may be located in only certain portions of the patch 130. For example, the adhesive 140 may be located along an outer perimeter of the patch 130. The patch 130 may be positioned on the retina with the retinal break in the center. Applying and/or having the adhesive 140 only along the outer perimeter may advantageously avoid the adhesive 140 from entering the sub-retinal space.

In some implementations, the adhesive 140 may be inoperative until it is activated. The adhesive 140 may be activated after the patch 130 is deployed within the vitreous chamber of the eye. For example, the adhesive 140 may be activated by and/or upon exposure to fluid, gel, and/or liquid, such as the vitreous humor, saline, balanced salt solution (BSS^{®}), balanced salt solution plus (BSS PLUS^{®}), water, and/or other suitable fluids. In additional examples, the adhesive 140 may be activated by and/or upon exposure to light. In some of these examples, the adhesive 140 may be activated by one or more wavelength(s) of light, including wavelength(s) of visible light, ultraviolet (UV) light, infrared (IR) light, etc. In some embodiments, the light is transmitted by a source remote from the eye, such as a treatment or diagnostic laser source or other light source, including incandescent light, halogen light, metal halide light, xenon light, mercury vapor light, light emitting diode (LED) light, other suitable sources, and/or combinations thereof. The light may be transmitted by an instrument positioned within the eye, such as an illumination device, laser probe, and/or photocoagulation probe. In some implementations, a curing agent of the patch 130 and/or the adhesive 140 may be activated by light or moisture.

The delivery device 102 is configured to deploy the patch 130 within a vitreous chamber of the eye. In some implementations, the delivery device 102 may be a disposable component that is designed for a single-use. In some implementations, the delivery device 102 is autoclavable and/or sterilizable such that the delivery device 102 may be reused. The delivery device 102 includes a body 110 and a cannula 120 that is coupled to the body 110. The body 110 may form a handle for the delivery device 102 and may be sized and shaped for handheld use and/or grasping by the user. The body 110 may be made of any desired or suitable material, such as a thermoplastic or metal. It may be formed by any method, including, for example, injection molding or machining. At least a portion of the body 110 may be knurled, patterned, and/or otherwise textured to improve gripping. While the illustrated implementation shows the body 110 to have a generally ellipsoidal shape, it is understood that the body 110 may be differently shaped in other implementations, including a tubular shape. Additionally, the body 110 may be formed of two or more sections that may be joined together. The size of the body 110 may also vary depending on the particular implementation.

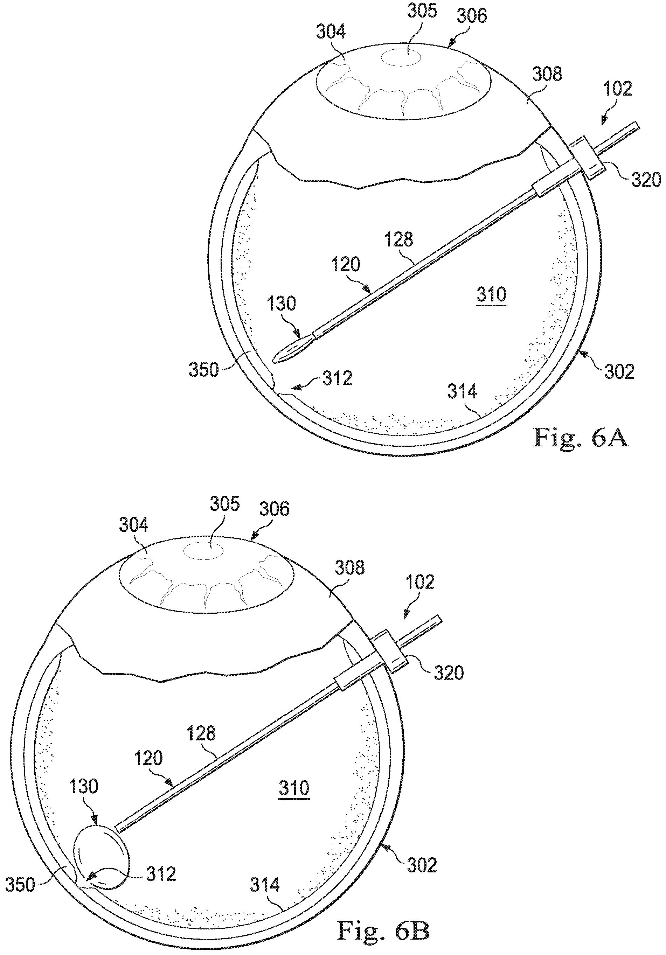

The cannula 120 includes a proximal portion 122, a distal portion 124, and a distal tip 126. The cannula 120 is sized and shaped for insertion into an interior space of the eye, such as the vitreous chamber. For example, the cannula 120 may be inserted into the eye through a trocar cannula 320, as illustrated in FIGs. 6A and 6B. The cannula 120 is also sized and shaped to accommodate the patch 130 in a furled or an unfurled state for passage through an incision in the eye. For example, in FIGs. 1 and 5A, the patch 130 is folded while positioned within the cannula 120. While the patch 130 is folded into thirds in the illustrated implementations, it is understood that the patch 130 may be unfolded, folded into two, three, four or more sections, rolled, and/or otherwise furled while disposed within the cannula 120.

The cannula 120 may be any suitable medical grade tubing, such as titanium, stainless steel, or suitable polymer. The cannula has a length 121 and a diameter 123. The length 121 of the cannula 120 may be between approximately 20 mm and approximately 40 mm, between approximately 20 mm and approximately 30 mm, between approximately 30 mm and approximately 40 mm, and/or other suitable values, both larger and smaller, in various implementations. The diameter of the cannula 120 may be between approximately 0.3 mm and approximately 1 mm, between approximately 0.3 mm and approximately 0.8 mm, between 0.5 mm and approximately 1 mm, and/or suitable values, both larger and smaller, in various implementations. In some implementations, the diameter 123 of the cannula 120 is constant along the length 121 while in other implementations the diameter varies. For example, the diameter 123 may increase or decrease from the proximal portion 122 to the distal portion 124 (or vice versa). In some embodiments, the cannula 120 may be cylindrically shaped so as to have a circular cross-section. In other embodiments, the cannula 120 may have a cross-section shaped as a polygon, an ellipse, other suitable shape, and/or a combination thereof.

The cannula 120 of the delivery device may be removably or fixedly coupled to the body 110. For example, the cannula 120 may be a disposable or single-use component while the body 110 is a sterilizable, multiple-use component. Different cannulas may be coupled to the same body 110 for different procedures. In other implementations, the cannula 120 may be fixedly coupled to the body 110. The delivery device 102, including cannula 120, may be autoclavable and/or otherwise sterilizable such that the delivery device may be used for multiple procedures. The delivery device 102, including the cannula 120, may be a disposable or single-use component.

A shaft 128 is movably disposed within the cannula 120 and may help eject the patch 130. For example, the shaft 128 may be actuatable such that the shaft 128 moves distally in direction 152 and/or proximally in direction 154. Movement of the shaft 128 within the cannula 120 can be free or continuous such that a distal tip 129 of the shaft 128 can be positioned in any number of locations relative to the distal tip 126 of the cannula 120. Movement of the shaft 128 within the cannula 120 can be constrained or gradated such that the distal tip 129 of the shaft 128 can be positioned in a fewer, finite number of locations relative to the distal tip 126 of the cannula 120. For example, the shaft 128 may be configured to move to one, two, three, four or more discrete positions. In an exemplary implementation, the distal tip moves between two positions. For example, before being actuated, the distal tip 129 of the shaft 128 may be disposed within the cannula 120 and may be spaced from the distal tip 126. The patch 130 may be disposed within the space between the distal tip 126 of the cannula 120 and the distal tip 129 of the shaft 128. When actuated, the shaft 128 may move distally in the direction 152, and may contact and push the patch 130 so as to eject the patch 130 from the cannula 120. The distal tip 129 of the shaft 128 may be suitably sized and shaped to contact and push the patch 130 without damaging or otherwise undesirably interfering with the patch 130. The distal tip 129 of the shaft 128 may be suitably sized and shaped or include features such as specific surface roughness to improve the ability to manipulate the patch 130. The distal tip 126 of cannula 120 may be suitably sized and shaped or include features such as specific surface roughness to improve the ability to manipulate the patch 130. When fully actuated, the distal tip 129 of the shaft 128 may be aligned with the distal tip 126 of the cannula 120 (FIGs. 5C, 5D).

Referring again to FIG. 1, a length 127 of the shaft 128 may be between approximately 20 mm and approximately 40 mm, between approximately 20 mm and approximately 30 mm, between approximately 30 mm and approximately 40 mm, and/or other suitable values, both larger and smaller, in various implementations. The length 127 of the shaft 128 may be equal to or greater than the length 121 of the cannula 120. For example, when the shaft 128 is fully actuated in the direction 152, the distal tip 129 of the shaft 128 may be aligned with or extend distally beyond the distal tip 126 of the cannula. At least a portion 119 of the shaft 128 may extend proximally into the body 110. The diameter 125 of the shaft 128 can be between approximately 0.1 mm and approximately 0.7 mm, between approximately 0.1 mm and approximately 0.4 mm, between approximately 0.4 mm and approximately 0.7 mm, and/or other suitable values, both larger and smaller, in various implementations. A diameter 125 of the shaft 128 may be less than the diameter 123 of the inner lumen of the cannula 120 such that the shaft 128 is able to move without contacting the walls of the cannula 120.

The system 100 may include a control mechanism 113 for actuating the shaft 128. The control mechanism 113 may be in mechanical, electrical, pneumatic, and/or otherwise suitable communication with the shaft 128. In some implementations, the control mechanism 113 for actuating the shaft 128 is integrated in the delivery device 102 as shown in FIG. 1. In the example shown, the delivery device 102 includes an actuation control 112, such as a button, a slider, and/or other suitable component that may cooperate with and may actuate the control mechanism 113. In the illustrated implementation, the actuation control 112 is disposed on or part of the body 110. The surgeon may act on the actuation control 112 with one or more fingers while grasping the body 110 with his or her hand. For example, the surgeon may press the button or move the slider in the direction 152 and/or the direction 154. For example, the slider may be moved in the direction 152 to eject the patch 130 from the cannula 120. The slider may be moved in the direction 154 to return the shaft 128 to an un-actuated position. In accordance with this, in some embodiments, the control mechanism 113 is a mechanical connection between the actuation control 112 and the shaft 128. Use of the actuation control 112 to eject the patch 130 from the cannula 120 is described in greater detail with respects to FIGs. 5A-5D.

In some implementations, the control mechanism 113 for actuating the shaft 128 is remote from the delivery device 102. For example, the control mechanism 113 may include a foot pedal or foot switch in communication with the shaft 128. Depression of the foot pedal or the foot switch may cause the shaft 128 to move in the direction 152 to eject the patch 130 from the cannula 120 and/or in the direction 154 to return the shaft 128 to an un-actuated position. In some instances, the control mechanism 113 may communicate pneumatic or electrical signals from a console to the delivery device 102.

In some implementations, one or more components of the system 100 may be integrated in a kit that is purchased by a hospital or other medical services provider. For example, the kit may include the patch 130, the delivery device 102, and the adhesive 140. The patch 130 may be disposed within the delivery device 102 such that user need only open the sealed kit and insert the delivery device 102 into the patient's eye to deploy the patch 130. The adhesive 140 may be integrated in the patch 130 or may be contained within a separate container. The kit may include a container having enough adhesive 140 for one patch 130. In some implementations, the applicator 340 (FIG. 6D) is included in the kit. The applicator 340 may be configured to obtain the adhesive 140 from the container and deliver the adhesive 140 to the retina and/or the patch 130.

FIGs. 5A-5D illustrate example functions of the ophthalmic surgical system 100, including the ejection of the patch 130 from the cannula 120. The functions discussed with respect to FIGs. 5A-5D may occur while the cannula 120 is at least partially positioned within the patient's eye. For example, FIGs. 6A and 6B, described in greater detail below, illustrate the cannula 120 and the patch 130 in situ in the eye. FIG. 5A illustrates the delivery device 102 and patch 130 just prior to being and/or once inserted into the patient's eye, according to an exemplary implementation. The patch 130 is disposed within the cannula 120 of the delivery device 102. FIG. 5A may also illustrate the delivery device 102 and the patch 130 after the user, such as a nurse or other medical professional, opens a sealed package from the manufacturer. In that regard, the patch 130 may be pre-loaded by the manufacturer within the cannula 120. In other implementations, the user may obtain the delivery device 102, the cannula 120, and/or the patch 130 separately, and thereafter load the patch 130 within the cannula 120. The patch 130 may be preformed in that the user receives the patch from a manufacturer ready for surgical use in a patient. For example, the size, shape, material, and/or other parameters of the patch 130 are pre-determined. In some implementations, the user may select an appropriately sized and shaped patch from among many patches depending on the size, shape, location, and other physiological parameters of the retinal break.

FIG. 5B illustrates the delivery device 102 as it is being actuated, according to an exemplary implementation. The surgeon may grasp the body 110 with his or her hand while maintaining or selectively positioning one or more fingers on the actuation control 112. In the illustrated implementation, the actuation control 112 may be a slider that is moved in the direction 152 by one or more of the user's fingers. Movement of the actuation control 112 causes corresponding movement of the shaft 128 in the distal direction 152. The actuation control 112 and the shaft 128 in FIG. 5B are positioned more distally than in FIG. 5A. Contact with the shaft 128 pushes the patch 130 more distally within the cannula 120, as illustrated in FIG. 5B when compared to FIG. 5A. FIG. 5B illustrates that the patch 130 is more than halfway ejected from the cannula 120 as a majority of the patch 130 extends beyond the distal tip 126 of the cannula 120. In some implementations, portions of the patch 130 begin to return to an unfurled state, such as by unrolling or unfolding, when the portions extend beyond the distal tip 126. In other implementations, the patch 130 begins to return to an unfurled state once the entire patch 130 extends beyond the distal tip 126.

FIG. 5C illustrates the delivery device 102 once it is fully actuated, according to an exemplary implementation. In that regard, the actuation control 112 and the shaft 128 may be at their farthest distal positions in the direction 152. In the illustrated implementation, the distal tip 129 of the shaft 128 is aligned with the distal tip 126 of the cannula 120. As a result of the contact with the shaft 128, the patch 130 is fully ejected from the cannula 120. The patch 130 is shown to be in the process of returning to an unfurled state in FIG. 5C. For example, the patch 130 is unfolding or unrolling itself because the walls of the cannula 120 no longer maintain the patch 130 in the furled state. FIG. 5D illustrates the patch 130 after it has unfolded or unrolled itself and fully returned to an unfurled state. In some implementations, the user may use cannula 120, the distal tip 126 of cannula 120, the distal tip 129 of the shaft 128, and/or the jaws 332 of forceps 330 (FIG. 6C) to ensure that the patch 130 returns to an unfurled state. In some implementations, the patch 130 may be delivered into the vitreous chamber of the eye in an unfurled state.

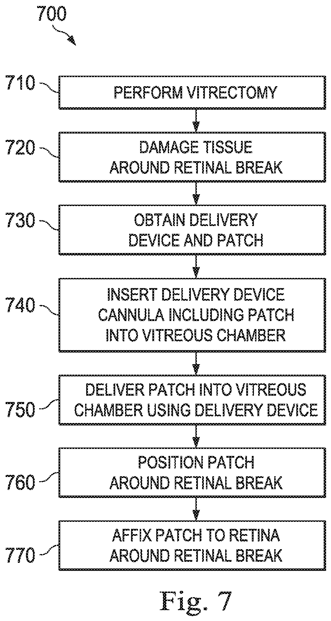

FIGs. 6A-6E illustrate components of the ophthalmic surgical system 100 in situ in the eye 302. Generally, the eye 302 includes an iris 304, a pupil 305, a cornea 306, a sclera 308, a vitreous chamber 310, a retina 314, and a sub-retinal space 350. The retina 314 includes a retinal break 312. FIGs. 6A-6E will be described in greater detail in the context of FIG. 7, which illustrates a flowchart of an example ophthalmic surgical method 700. As illustrated, the method 700 includes a number of enumerated steps, but implementations of the method 700 may include additional steps before, after, and in between the enumerated steps. In some implementations, one or more of the enumerated steps may be omitted or performed in a different order.

At 710, the method 700 includes performing a vitrectomy procedure. The vitrectomy procedure removes a portion of the vitreous humor from the patient's eye. The vitrectomy procedure may involve one or more tools positioned within the patient's eye 302. The method 700 may include inserting an illumination device, an infusion cannula, a vitrectomy probe, an aspiration probe, and/or other suitable device(s) into the patient's eye 302. The user may create incisions through the sclera 308 in the pars plana using a trocar. The incision is called a sclerotomy. A trocar blade is then removed, with the trocar cannula 320 remaining within the incision and defining a lumen into the posterior segment of the eye, such as the vitreous chamber 310. For simplicity, only one port or trocar cannula 320 that facilitates access to the vitreous chamber 310 is illustrated in FIGs. 6A-6D. It is understood that two, three, four or more ports or trocar cannulas may positioned in the sclera 308 to allow multiple tools to be positioned within the eye 302 during the surgical procedure. During the procedure, while viewing the posterior segment under a microscope and with the aid of an illumination device, the surgeon cuts and aspirates away vitreous or other tissue using the vitrectomy probe to gain access to the area of interest (e.g., the site of the retinal detachment, tear, hole, or break 312). Vitrectomy may also remove vitreous that is the source of the traction causing the retinal break.

Referring again to FIG. 7, at 720, the method 700 includes destroying or damaging tissue around the retinal break 312 (FIG. 6A). Destroying or damaging tissue can include performing a laser retinopexy/photocoagulation or cryopexy. Laser retinopexy/photocoagulation utilizes pulses of light and cryopexy utilizes intense cold to destroy or damage tissue around the retinal break 312. As the damaged tissue heals, scar tissue develops around the retinal break 312 over the course of time and seals the retinal break 312. In some implementations, the method 700 can include a fluid/air exchange, during which the fluid (e.g., vitreous humor, etc.) from the eye 302 is evacuated and filled with air to maintain intraocular pressure. Laser retinopexy/photocoagulation or cryopexy may be performed while the eye 302 is filled with air.

Referring again to FIG. 7, at 730, the method 700 includes obtaining the delivery device 102 and the patch 130 (FIG. 6A). The patch 130 is disposed within the cannula 120 of the delivery device 102 in a folded, rolled, and/or otherwise furled state. At 740, the method 700 includes inserting the cannula 120 and the patch 130, disposed within the cannula 120, into the eye 302. As shown in FIG. 6A, the cannula 120 of the delivery device 102 is inserted through the trocar cannula 320 into the vitreous chamber 310. At 750, the method 700 includes delivering the patch 130 into the vitreous chamber 310, using the delivery device 102 (FIGs. 6A, 6B). In some implementations, the patch 130 is delivered into the eye 302 while the eye 302 is filled with air, such as after fluid/air exchange. Delivering the patch 130 can include actuating the shaft 128 of the delivery device 102 to contact and push the patch 130 out of the cannula 120. For example, the user may depress a button, slide a slider, and/or otherwise engage the actuation control 112 on the delivery device 102 (FIGs. 1, 5A-5D). In other implementations, the user may depress a footswitch and/or otherwise engage a control mechanism to actuate the shaft 128. FIG. 6A illustrates the shaft 128 pushing the patch 130 to eject the patch 130 from the cannula 120. FIG. 6B illustrates the patch 130 after it has been ejected from the cannula 120 and after the patch 130 assumes an unfolded, unrolled, and/or otherwise unfurled state. In some implementations, the method 700 may include the user unfurling the patch 130, such as by manipulating the patch 130 using the cannula 120 and/or the forceps 330 (FIG. 6C) to unroll or unfold the patch 130. In some implementations, the method 700 can include removing the delivery device 102 from the eye 302.

Referring again to FIG. 7, at 760, the method 700 includes positioning the patch 130 around the retinal break 312. Positioning the patch 130 can include moving, orienting, and/or otherwise manipulating the patch 130 so that the patch 130 is in contact with the retina 314 and centered on the retinal break 312. For example, the patch 130 may be positioned to cover and seal the retinal break 312. In some implementations, the cannula 120 can be used to position the patch 130. In other implementations, a separate instrument, such as the forceps 330, may be inserted into the vitreous chamber 310 (FIG. 6C). The user may utilize the jaws 332 of the forceps 330 to grab and manipulate the patch 130 as necessary. In that regard, the forceps 330 may be considered part of the ophthalmic surgical system 100 (FIG. 1).

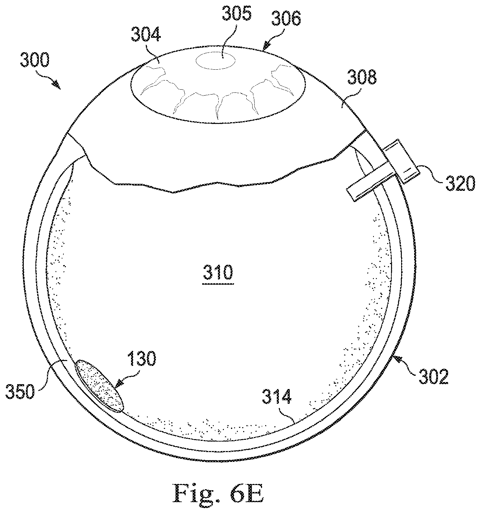

Referring again to FIG. 7, at 770, the method 700 includes affixing the patch 130 to the retina 314. Affixing the patch 130 may include applying the adhesive 140 to the patch 130 and/or the retina 314 to seal the retinal break 312 (FIG. 6D). The applicator 340 may be inserted into the vitreous chamber 310. The applicator 340 may dispense the adhesive 140 from a distal tip 342. In some implementations, the adhesive 140 may be distributed along a perimeter of the patch 130. In some implementations, the adhesive 140 may be distributed across the entire surface of the patch. In some implementations, the adhesive 140 may extend onto the surface of the retina. In implementations in which the adhesive 140 is disposed on or within the patch 130, the patch 130 may be affixed to the retina 314 without the applicator 340 (FIG. 6E). In some implementations, the patch 130 may be exposed to fluid or light to activate the adhesive 140. For example, fluid may be returned to the vitreous chamber 310 in an air/fluid exchange. The adhesive 140 may be activated once the vitreous chamber 310 is filled with fluid. In other implementations, the user may inject saline, BSS^{®}, or BSS PLUS^{®} onto the patch 130 while the vitreous chamber 310 is still filled with air. In other implementations, the adhesive 140 may be activated by contact with fluid already present on the retina or the wetness of the retina itself. As a result, the adhesive 140 is activated and the patch 130 is affixed to the retina 314 before the vitreous humor is reintroduced into the vitreous chamber 310. In another example, light having a particular wavelength, such as laser light and/or other suitable light, may be directed to the patch 130 to activate the adhesive 140. The laser light may be delivered from a laser source outside of the eye 302. In other implementations, light composed of many wavelengths or a spectrum of wavelengths, for example white light, may be directed to the patch 130 to activate the adhesive 140. In some implementations, the adhesive 140 may be able to be activated by a plurality of wavelengths. The light may be delivered from an instrument, such as a photocoagulation probe or illumination probe, disposed within the eye 302. The patch 130 is illustrated with texture to indicate that the adhesive has been used to affix the patch 130 to the retina 314 and/or that the adhesive has been activated. In some implementations, the user may hold the patch 130 in the desired position using the cannula 120 or the forceps 330 while the patch 130 is being affixed to the retina 314. Affixing the patch 130 to the retina 314 seals the retinal break 312 and prevents fluid from entering the sub-retinal space 350. In some implementations, the method 700 includes sealing the retinal break 312 without injecting oil/gas into the eye 302 to seal the retinal break 312. The method 700 may also include sealing the retinal break 312 without positioning the patient so that the oil/gas presses against and seals the retinal break 312.

It should be understood that all references and discussion relating to the patch 130, including the method 700 of implanting the patch 130, may equally apply to any of the other patch embodiments described herein.

Persons of ordinary skill in the art will appreciate that the implementations encompassed by the present disclosure are not limited to the particular exemplary implementations described above. In that regard, although illustrative implementations have been shown and described, a wide range of modification, change, combination, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An ophthalmic surgical system (100), comprising:
a patch (130) sized and shaped to seal a retinal break of an eye by preventing fluid from infiltrating a sub-retinal space when adhered to a retina surrounding the retinal break, wherein the patch (130) is sized and shaped according to a size and shape of the retinal break, and wherein the patch (130) is formed of a material that does not break down or degrade over time so that the patch is permanently positioned around the retinal break;
a delivery device (102) including a cannula (120) configured to maintain the patch in a furled state for passage through an incision in the eye, the delivery device actuatable to deploy the patch within a vitreous chamber of the eye; and
a biocompatible adhesive (140), disposed on the patch, to affix the patch to the retina.

2. The system of claim 1, wherein the patch (130) comprises at least one of a hydrogel, a biological material, an elastomeric polymer, a cross-linking agent, or a curing agent.

3. The system of claim 1, wherein the adhesive (140) is activated upon exposure to liquid.

4. The system of claim 1, wherein the adhesive (140) is activated upon exposure to light.

5. The system of claim 1, further comprising an applicator (340) sized and shaped for insertion into the vitreous chamber, the applicator configured to apply the adhesive to at least a perimeter of the patch while the patch is positioned on the retina.

6. The system of claim 1, wherein the delivery device (102) further comprises a body (110) coupled to the cannula, the body being sized and shaped for grasping by a user.

7. The system of claim 6, wherein the delivery device (102) includes a shaft (128) movably disposed within the cannula, the shaft configured to eject the patch from the cannula upon movement of the shaft.

8. The system of claim 7, wherein the shaft (128) is coupled to an actuation control (112) controlling movement of the shaft.

9. The system of claim 8, wherein the actuation control (112) is disposed on the body (110) of the delivery device.

10. The system of claim 1, wherein the delivery device (102) comprises a body (110), the cannula (120) coupled to the body (110), and a shaft (128) movably disposed within the cannula, the cannula being sized and shaped to be inserted into a vitreous chamber of the eye; and
wherein the patch (130) is disposed within the cannula in a furled state, and wherein, upon actuation of the shaft (128), the patch is ejected from the cannula into the vitreous chamber, the patch being adjustable to an unfurled state.

## Patentansprüche

1. Augenchirurgisches System (100), umfassend:
einen Patch (130), der zum Versiegeln eines Netzhautbruchs eines Auges bemessen und gestaltet ist, indem er verhindert, dass Fluid in einen subretinalen Raum eindringt, wenn er an einer den Netzhautbruch umgebenden Netzhaut haftet, wobei der Patch (130) gemäß einer Größe und Gestalt des Netzhautbruchs bemessen und gestaltet ist und wobei der Patch (130) aus einem Material ausgebildet ist, das sich im Laufe der Zeit weder zersetzt noch abbaut, so dass der Patch permanent um den Netzhautbruch herum positioniert ist;
eine Zuführvorrichtung (102), die eine Kanüle (120) aufweist, die dazu ausgestaltet ist, den Patch für den Durchgang durch eine Inzision in dem Auge in einem zusammengefächerten Zustand zu halten, wobei die Zuführvorrichtung betätigbar ist, um den Patch in einem Glaskörperraum des Auges auszubringen; und
einen auf dem Patch angeordneten biokompatiblen Klebstoff (140) zum Befestigen des Patch an der Netzhaut.

2. System nach Anspruch 1, wobei der Patch (130) ein Hydrogel und/oder ein biologisches Material und/oder ein elastomeres Polymer und/oder einen Vernetzer und/oder einen Härter umfasst.

3. System nach Anspruch 1, wobei der Klebstoff (140) aktiviert wird, wenn er Flüssigkeit ausgesetzt wird.

4. System nach Anspruch 1, wobei der Klebstoff (140) aktiviert wird, wenn er Licht ausgesetzt wird.

5. System nach Anspruch 1, ferner umfassend einen Applikator (340), der für die Einführung in den Glaskörperraum bemessen und gestaltet ist, wobei der Applikator dazu ausgestaltet ist, den Klebstoff auf mindestens einen Umfang des Patch aufzubringen, während der Patch auf der Netzhaut positioniert ist.

6. System nach Anspruch 1, wobei die Zuführvorrichtung (102) ferner einen an die Kanüle gekoppelten Körper (110) umfasst, wobei der Körper so bemessen und gestaltet ist, dass er von einem Benutzer ergriffen werden kann.

7. System nach Anspruch 6, wobei die Zuführvorrichtung (102) einen Schaft (128) aufweist, der beweglich in der Kanüle angeordnet ist, wobei der Schaft dazu ausgestaltet ist, den Patch bei Bewegung des Schafts aus der Kanüle auszustoßen.

8. System nach Anspruch 7, wobei der Schaft (128) an eine Betätigungssteuerung (112) gekoppelt ist, die die Bewegung des Schafts steuert.

9. System nach Anspruch 8, wobei die Betätigungssteuerung (112) an dem Körper (110) der Zuführvorrichtung angeordnet ist.

10. System nach Anspruch 1, wobei die Zuführvorrichtung (102) einen Körper (110), die an den Körper (110) gekoppelte Kanüle (120) und einen beweglich in der Kanüle angeordneten Schaft (128) umfasst, wobei die Kanüle für die Einführung in einen Glaskörperraum des Auges bemessen und gestaltet ist; und
wobei der Patch (130) in einem zusammengefächerten Zustand in der Kanüle angeordnet ist und wobei der Patch bei Betätigung des Schafts (128) aus der Kanüle in den Glaskörperraum ausgestoßen wird, wobei der Patch zu einem aufgefächerten Zustand verstellbar ist.

## Revendications

1. Système chirurgical ophtalmique (100), comprenant :
un timbre (130) dimensionné et formé pour sceller une déchirure rétinienne d'un œil en empêchant un fluide de s'infiltrer dans un espace sous-rétinien lorsqu'il adhère à une rétine entourant la déchirure rétinienne, le timbre (130) étant dimensionné et formé selon une taille et une forme de la déchirure rétinienne, et le timbre (130) étant formé d'un matériau qui ne se décompose pas ou ne se dégrade pas avec le temps de sorte que le timbre soit positionné de manière permanente autour de la déchirure rétinienne ;
un dispositif de pose (102) comprenant une canule (120) configurée pour maintenir le timbre dans un état enroulé pour un passage à travers une incision dans l'œil, le dispositif de pose pouvant être actionné pour déployer le timbre dans une chambre vitrée de l'œil ; et
un adhésif biocompatible (140), disposé sur le timbre, pour fixer le timbre à la rétine.

2. Système selon la revendication 1, dans lequel le timbre (130) comprend au moins l'un d'un hydrogel, d'un matériau biologique, d'un polymère élastomère, d'un agent de réticulation, ou d'un agent de durcissement.

3. Système selon la revendication 1, dans lequel l'adhésif (140) est activé par exposition à un liquide.

4. Système selon la revendication 1, dans lequel l'adhésif (140) est activé par exposition à la lumière.

5. Système selon la revendication 1, comprenant en outre un applicateur (340) dimensionné et formé pour être inséré dans la chambre vitrée, l'applicateur étant configuré pour appliquer l'adhésif sur au moins un périmètre du timbre pendant que le timbre est positionné sur la rétine.

6. Système selon la revendication 1, dans lequel le dispositif de pose (102) comprend en outre un corps (110) accouplé à la canule, le corps étant dimensionné et formé pour être saisi par un utilisateur.

7. Système selon la revendication 6, dans lequel le dispositif de pose (102) comprend une tige (128) disposée de manière mobile à l'intérieur de la canule, la tige étant configurée pour éjecter le timbre de la canule lors d'un mouvement de la tige.

8. Système selon la revendication 7, dans lequel la tige (128) est accouplée à une commande d'actionnement (112) commandant un mouvement de la tige.

9. Système selon la revendication 8, dans lequel la commande d'actionnement (112) est disposée sur le corps (110) du dispositif de pose.

10. Système selon la revendication 1, dans lequel le dispositif de pose (102) comprend un corps (110), la canule (120) accouplée au corps (110), et une tige (128) disposée de manière mobile à l'intérieur de la canule, la canule étant dimensionnée et formée pour être insérée dans une chambre vitrée de l'œil ; et
dans lequel le timbre (130) est disposé à l'intérieur de la canule dans un état enroulé, et dans lequel, lors de l'actionnement de la tige (128), le timbre est éjecté de la canule dans la chambre vitrée, le timbre pouvant être ajusté vers un état déroulé.
